**Europäisches Patentamt**

**European Patent Office**

**Office européen des brevets**

(19)

(11) Veröffentlichungsnummer : **0 088 860**
**B1**

(12) # EUROPÄISCHE PATENTSCHRIFT

(45) Veröffentlichungstag der Patentschrift :
10.09.86

(51) Int. Cl.⁴ : **A 61 F 15/00**

(21) Anmeldenummer : **82890038.1**

(22) Anmeldetag : **12.03.82**

(54) **Vorrichtung zum Feststellen des Druckes zwischen einem Stützverband und einem von diesem Stützverband umgebenen Körperteil.**

(43) Veröffentlichungstag der Anmeldung :
**21.09.83 Patentblatt 83/38**

(45) Bekanntmachung des Hinweises auf die Patenterteilung : **10.09.86 Patentblatt 86/37**

(84) Benannte Vertragsstaaten :
**BE CH DE FR GB IT LI NL SE**

(56) Entgegenhaltungen :
DE-A- 2 911 912
DE-C-   363 101
FR-A-   985 553
FR-A- 2 086 989
US-A- 3 998 220
US-A- 4 286 603
US-A- 4 331 133

(73) Patentinhaber : **Ender, Hans Georg, Dr.**
**Krenngasse 3**
**A-1180 Wien (AT)**

(72) Erfinder : **Ender, Hans Georg, Dr.**
**Fersteigasse 6/20**
**A-1090 Wien (AT)**
Erfinder : **Sillaba, Wilhelm**
**Kaisermühlendamm 103/6**
**A-1223 Wien (AT)**

(74) Vertreter : **Boeckmann, Peter, Dipl.Ing. et al**
**Strohgasse 10**
**A-1030 Wien (AT)**

## Beschreibung

Die Erfindung betrifft eine Vorrichtung zum Feststellen des Druckes zwischen einem Stützverband und einem von diesem Stützverband umgebenen Körperteil, mit einem an diesem Körperteil anliegenden und am Stützverband abgestützten Druckfühler, der mit einer Druckanzeigevorrichtung gekuppelt ist.

Bei bestimmten Verletzungen, wie Knochenbrüchen, Sehnenzerrungen od. dgl. wird der betroffene Körperteil mit einem Stützverband umgeben, um eine Schienung und Ruhigstellung dieses Körperteiles bzw. eine Heilung des Knochenbruches in der richtigen Lage der Fragmente an der Bruchstelle sicherzustellen. Als Stützverband wird in der Regel ein Gipsverband oder ein anderer starrer Verband verwendet, der eine entsprechende Schienung des betreffenden Körperteiles bewirkt. Nachteilig bei dieser Vorgangsweise ist nur, daß dieser Körperteil in der Regel in der ersten Zeit nach dem Anlegen des Stützverbandes anschwillt, wobei die Schwellung einen solchen Wert annehmen kann, daß dieser Körperteil durch den unnachgiebigen Stützverband so stark abgeschnürt wird, daß eine irreversible Störung, im Extremfall sogar ein Absterben des Körperteiles hervorgerufen wird.

Um diesem Nachteil zu begegnen, ist es bekannt, den Stützverband zunächst zu spalten und bei Auftreten der Schwellung an der Spaltstelle aufzubiegen, damit der angeschwollene Körperteil im Stützverband Platz finden kann, und erst nach Ablauf der Schwellzeit einen geschlossenen Gipsverband anzulegen. Durch einen solchen gespaltenen Gipsverband wird jedoch wieder der Nachteil erzielt, daß eine unzureichende Unterstützung des betreffenden Körperteiles, insbesondere dann, wenn die Schwellung nicht im erwarteten Maße auftritt oder frühzeitig abklingt, gegeben ist. Es können sich dann die Fragmente nachträglich an der Bruchstelle verschieben und es ist die gewünschte Heilung des Knochenbruches nicht mehr gewährleistet.

Es ist weiters bekannt, zwischen dem Stützverband und dem von diesem umgebenen Körperteil Watte oder andere komprimierbare Materialien einzulegen, welche bei Auftreten der Schwellungen zusammengedrückt werden. Auch hier tritt der Nachteil auf, daß dann, wenn die Schwellung nicht im erwarteten Maße auftritt bzw. frühzeitig zurückgeht, der betreffende Körperteil unzureichend unterstützt ist, die Schwellung aber auch solche Werte annehmen kann, daß die komprimierbare Einlage nicht mehr in der Lage ist, die durch die Schwellung verursachte Vergrößerung des Körperteiles zu kompensieren.

In jedem Falle muß dann, wenn die Schwellung so groß wird, daß wesentliche Durchblutungsstörungen des betreffenden Körperteiles auftreten, der Arzt Abhilfe schaffen. Es ist nun schwierig vom betreffenden Patienten zu erkennen, wann die Schwellungen ein solches Maß angenommen haben, daß eine gefährliche Situation besteht, oder wann die Schwellungen noch tolerable Werte besitzen. Abgesehen davon, daß die durch die Schwellung hervorgerufenen Schmerzen von den einzelnen Patienten verschieden beurteilt werden und daher hier den Patienten keine genauen Richtlinien gegeben werden können, sind beispielsweise alte oder bewußtlose Personen nicht in der Lage, auf auftretende Schmerzen entsprechend zu reagieren.

Es ist auch bekannt, zwischen dem Stützverband und dem von diesem umgebenen Körperteil Kapseln einzubetten, die eine farbige Flüssigkeit einschließen und die bei Auftreten eines bestimmten Druckes platzen, wodurch sich der Gipsverband verfärbt.

Eine ähnliche Vorrichtung offenbart die US-A 4 286 603. Diese Vorrichtung besteht aus einer teilweise flexiblen Kapsel, die zum Teil in den Stützverband eingebettet ist und mit einem Ansatz aus dem Stützverband herausragt oder zumindest bis zur äußeren Oberfläche des Stützverbandes reicht. Die Kapsel ist mit einer Anzeigeflüssigkeit gefüllt, wobei entweder der die Anzeigeflüssigkeit enthaltende Teil der Kapsel vom Ansatz durch eine zerbrechliche Scheibe getrennt ist, oder sich im Inneren der Kapsel eine mit der Anzeigeflüssigkeit gefüllte Blase befindet. Die Anzeige einer unzulässigen Drucküberschreitung erfolgt dadurch, daß bei einer solchen Drucküberschreitung die zerbrechliche Scheibe oder die Blase zerstört wird und in der Folge die Anzeigeflüssigkeit in den Ansatz gedrängt wird und dort sichtbar wird, wobei der Ansatz mit Markierungen versehen sein kann. Die Kapsel weist entweder zumindest an jener Stelle, an der sie am zu stützenden Körperteil anliegt, eine nachgiebige Wand auf oder ist dort mit einer im wesentlichen starren Wand versehen, besitzt jedoch in diesem Fall als Faltenbalg ausgebildete Seitenwände und ist dann in einem starren Becher aufgenommen. Auch in diesem Fall erfolgt die Anzeige dadurch, daß eine zerbrechliche Scheibe im Inneren der Kapsel bei einer unzulässigen Drucksteigerung bricht und dadurch die Anzeigeflüssigkeit in den Ansatz fließt. Da die Anzeige einer unzulässigen Drucksteigerung bei dieser bekannten Vorrichtung durch Zerstörung der im Inneren der Kapsel befindlichen, mit einer Anzeigeflüssigkeit gefüllten Blase oder durch Zerstörung der zerbrechlichen Scheibe im Inneren der Kapsel erfolgt, kann diese bekannte Vorrichtung nur einmal verwendet werden und ist nach Zerstörung der erwähnten Teile nicht mehr einsatzfähig. Ein wesentlicher Nachteil besteht weiters darin, daß es mit dieser bekannten Vorrichtung nicht möglich ist, Druckschwankungen anzuzeigen, sondern diese bekannte Vorrichtung läßt lediglich eine einmalige Überschreitung eines bestimmten Druckwertes erkennen. Die Anzeige von Druckschwankungen ist jedoch deshalb wesentlich, da es häufig genügt, den betreffenden Körperteil nach Überschreiten des gefährlichen Druckwertes hochzulagern, ohne am Stützverband selbst etwas zu verändern, worauf der Druck wieder tolerierbare Werte annimmt.

Schließlich ist es bekannt, zwischen dem Stützverband und dem von diesem umgebenen Körperteil einen elektrischen Kondensator anzuordnen, dessen Dilectricum aus einem zusammendrückbaren Kunststoffschaum besteht, so daß bei einer Änderung der Druckverhältnisse zwischen dem Stützverband und dem Körperteil die Kapazität dieses Kondensators verändert wird. Die Kondensatorplatten sind nun über eine elektrische Leitung mit einer Auswertungsanlage verbunden, die gesondert vom Patienten aufgestellt ist. Bei Anwendung dieser bekannten Vorrichtung kann sich der Patient somit nicht bewegen, sondern ist vielmehr an das Bett oder an einen Stuhl gefesselt, da er ja über die elektrischen Leitungen mit der Auswerteeinrichtung verbunden ist. Diese Vorrichtung ist daher ausschließlich für den Einsatz in Spitälern gedacht. Nun werden aber bei Knochenbrüchen, Sehnenzerrungen od. dgl. die Patienten zum überwiegenden Teil nach Anlegen des Stützverbandes nach Hause entlassen, wobei die Gefahr einer Schwellung in der ersten Zeit nach der Entlassung auftritt. Um in dieser Zeit einen unzulässigen Druckanstieg festzustellen, ist diese bekannte Vorrichtung nicht geeignet.

Die vorliegende Erfindung hat sich zur Aufgabe gestellt, eine Vorrichtung zum Feststellen des Druckes zwischen einem Stützverband und einem von diesem Stützverband umgebenen Körperteil zu schaffen, die einfach in ihrem Aufbau ist, ein geringes Volumen aufweist, auch Druckschwankungen anzeigt und mehrfach verwendet werden kann, wobei eine Weiterverwendung bereits dann möglich ist, wenn der Stützverband noch angelegt ist, jedoch die Gefahr einer Schwellung des Körperteiles und daher eines unzulässigen Druckanstieges nicht mehr gegeben ist. Zur Lösung dieser Aufgabe wird erfindungsgemäß vorgeschlagen, daß der Druckfühler und vorzugsweise auch die Druckanzeigevorrichtung in einem Gehäuse angeordnet sind, welches in einem im Stützverband unverrückbar verankerbaren Haltekörper entfernbar eingesetzt ist und daß der am Körperteil unmittelbar oder mittelbar anlegbare Druckfühler so ausgebildet ist, daß der Druck mechanisch und/oder mittels eines Druckmittels auf die Druckanzeigevorrichtung übertragbar ist. Eine solche Ausbildung der erfindungsgemäßen Vorrichtung weist mehrere Vorteile auf. So ist es möglich, beim Anlegen des Stützverbandes zunächst nur den Haltekörper im Stützverband zu verankern und erst nach dem Erhärten des beispielsweise als Gipsverband ausgebildeten Stützverbandes Druckfühler und Druckanzeigevorrichtungen in den Haltekörper einzusetzen und dort zu fixieren. Dadurch werden Beschädigungen, Verschmutzungen und dgl. des Druckfühlers und der Druckanzeigevorrichtung beim Anlegen des Stützverbandes vermieden und es wird verhindert, daß durch Eindringen von flüssigem Gips die Vorrichtung nicht mehr die richtigen Werte anzeigt. Außerdem kann bei einer solchen Ausführungsform auf einfache Weise eine Justierung der Druckanzeigevorrichtung dadurch vorgenommen werden, daß die Lage des Druckfühlers im Haltekörper verändert wird. Da die erfindungsgemäße Vorrichtung stets den momentan auftretenden Druckwert anzeigt, kann der Druckverlauf ohne Schwierigkeiten beobachtet werden, es kann also der Erfolg verschiedener Maßnahmen zum Abklingen der einen Anstieg des Druckes bewirkenden Schwellung des Körperteiles festgestellt werden. Schließlich ist es bei dieser Ausführungsform möglich, Druckfühler und Druckanzeigevorrichtung nach dem Abklingen der Schwellung auf einfache Weise aus dem Haltekörper auszubauen, so daß diese Geräte wieder bei einem anderen Patienten zum Einsatz gebracht werden können, ohne daß der Stützverband beim Ausbau beschädigt wird. Es verbleibt dann lediglich der Haltekörper im Stützverband.

Um eine sichere und unverrückbare Verankerung des Haltekörpers im Stützverband zu gewährleisten, was für die einwandfreie Funktionsweise der erfindungsgemäßen Vorrichtung wesentlich ist, kann erfindungsgemäß dieser Haltekörper an seiner Außenseite mit Vorsprüngen, wie Rippen, Flügeln od. dgl. versehen sein, welche nach dem Erhärten des Stützverbandes eine sichere Verbindung zwischen diesem und dem Haltekörper gewährleisten.

Gemäß einer vorteilhaften Ausführungsform der Erfindung ist der Haltekörper von einer Hülse gebildet, die zumindest teilweise mit einem Innengewinde versehen ist, und es ist das Gehäuse zylindrisch ausgebildet und weist ein dem Innengewinde der Hülse entsprechendes Außengewinde auf. Eine solche Ausbildung ermöglicht eine einfache und sichere Verankerung des Gehäuses im Haltekörper, da das Gehäuse nur in den Haltekörper eingeschraubt werde braucht. Bei einer solchen Ausführungsform läßt sich aber auch die Justierung der erfindungsgemäßen Vorrichtung leicht dadurch vornehmen, daß das Gehäuse so weit in die Hülse eingeschraubt wird, bis die Druckanzeigevorrichtung den vorbestimmten Normwert anzeigt. Durch Verwendung eines Feingewindes läßt sich die Justierung leichter durchführen.

Um sicherzustellen, daß der eingestellte Normwert nicht nachträglich durch Verdrehung des Gehäuses beispielsweise durch den Patienten verändert wird, ist erfindungsgemäß auf das Gehäuse eine Gegenmutter zur Fixierung seiner Lage in Bezug auf die Hülse aufgeschraubt. Es kann aber auch im Gehäuse und/oder in der Hülse eine Fixierschraube vorgesehen sein, welche zur Fixierung der eingestellten Lage dient.

Befindet sich das Gehäuse nicht im Haltekörper, so ist es von Vorteil, wenn im Haltekörper ein Füllkörper eingesetzt ist. Dieser Füllkörper verhindert ein Eindringen von Schmutz und Fremdkörpern in den Haltekörper, wodurch das Gewinde des Haltekörpers verunreinigt wird und unter Umständen eine Infektion hervorgerufen werden kann. Besonders beim Anlegen des Stützverbandes ist es wesentlich, daß der hiebei in den Stützverband einzubettende Haltekörper abgeschlossen ist, da sonst der Gips oder das andere erhärtende Material in das Innere des Haltekörpers eindringt und dann mühsam entfernt werden müßte. Aber auch nach dem Entfernen des Druckfühler und Druckanzeigevorrichtung aufnehmenden Gehäuses aus dem Haltekörper nach dem Abklingen der Schwellung wird der Haltekörper zweckmäßig mittels des Füllkörpers verschlossen.

Der Druckfühler kann erfindungsgemäß von einem im Gehäuse verschiebbar angeordneten Einsatzteil gebildet sein, der über ein Gestänge od. dgl. mit einem Zeiger verbunden ist, der zusammen mit einer Skala die Druckanzeigevorrichtung bildet. Der verschiebbar angeordnete Einsatzteil kann hiebei durch eine Feder od. dgl. oder durch ein Druckmittel hydraulisch in seine Ruhestellung gedrückt sein.

Liegt der Einsatzteil direkt am betreffenden Körperteil an, so werden nur die senkrecht auf diesen Einsatzteil einwirkenden Drücke exakt übertragen und ermittelt bzw. es können schräg auf den Einsatzteil einwirkende Drücke zu dessen Verklemmen im Gehäuse führen, wodurch die Vorrichtung funktionsfähig wird. Um dies zu vermeiden, ist gemäß einem weiteren Merkmal der Erfindung der verschiebbar angeordnete Einsatzteil mit einem mit einem unkomprimierbaren Medium gefüllten elastischen Körper verbunden, der am Körperteil anliegt. Dieser elastische Körper paßt sich der Form des betreffenden Körperteiles an und gewährleistet eine exakte Übertragung der auftretenden Drücke unabhängig von ihrer Richtung.

Bei einer anderen Ausführungsform der erfindungsgemäßen Vorrichtung ist im Gehäuse ein teilweise am Körperteil anliegender elastischer Körper angeordnet, der mit einem unkomprimierbaren Medium gefüllt ist und über eine Leitung mit der Druckanzeigevorrichtung verbunden ist. Eine solche Ausführungsform ermöglicht ohne mechanisches Hebelsystem durch das unkomprimierbare Medium eine Übertragung des auftretenden Druckes an die Druckanzeigevorrichtung. Diese Druckanzeigevorrichtung kann beispielsweise aus einem mit der Leitung verbundenen Schauglas und einer neben dem Schauglas angeordneten Skala bestehen, in welchem Fall der im Schauglas auftretende Flüssigkeitsspiegel dem Wert des auftretenden Druckes entspricht, welcher auf der Skala abgelesen werden kann.

Bei einer anderen Ausführungsform kann ein in einem zumindest teilweise mit einem unkomprimierbaren Medium gefüllten Zylinder angeordneter Kolben vorgesehen sein, dessen Kolbenstange mit einem Zeiger in Verbindung steht, der mit einer Skala zusammenwirkt. Der Zylinder kann hiebei beispielsweise von einer elastischen bzw. nachgiebigen Wand begrenzt sein, die am Körperteil anliegt, so daß sich der Druck über diese nachgiebige Wand und das unkomprimierbare Medium auf den Kolben überträgt und dieser bei einer Änderung des Druckes bewegt wird. Es kann aber auch der Zylinderraum über eine Leitung mit einem teilweise am Körperteil anliegenden elastischen Körper verbunden sein, der mit einem unkomprimierbaren Medium gefüllt ist, so daß sich auch hiedurch eine Verschiebung des Kolbens bei einer Änderung der Druckverhältnisse ergibt.

Die Skala ist vorzugsweise in drei Bereiche unterteilt. Hier sind verschiedene Möglichkeiten gegeben. Vorteilhaft ist es, wenn der mittlere Bereich jenen Bereich darstellt, in welchem der Druck tolerierbare Werte aufweist. Die durch den daran anschließenden oberen Bereich definierten Drücke ergeben bereits eine Gefahr für den Patienten, es muß daher bei Auftreten dieser Drücke vom Arzt Abhilfe geschaffen werden. Treten hingegen Drücke in dem an den mittleren Bereich anschließenden unteren Bereich auf, so ist der Stützverband zu locker geworden und kann daher seine Stützfunktion nicht mehr ausüben. Auch in diesem Fall sind Gegenmaßnahmen des Arztes erforderlich.

Es können aber auch die drei Bereiche so eingeteilt sein, daß der unterste Bereich den normalerweise auftretenden Drücken entspricht, die durch den daran anschließenden Übergangsbereich definierten Drücke gerade noch tolerierbar sind und die in dem an diesen Übergangsbereich anschließenden Bereich auftretenden Drücke eine unmittelbare Gefahr für den Patienten darstellen.

Die erfindungsgemäße Vorrichtung ermöglicht somit auch die Feststellung einer unzulässigen Lockerung des Stützverbandes, da dann der gemessene Druck unter den Sollwert abfällt.

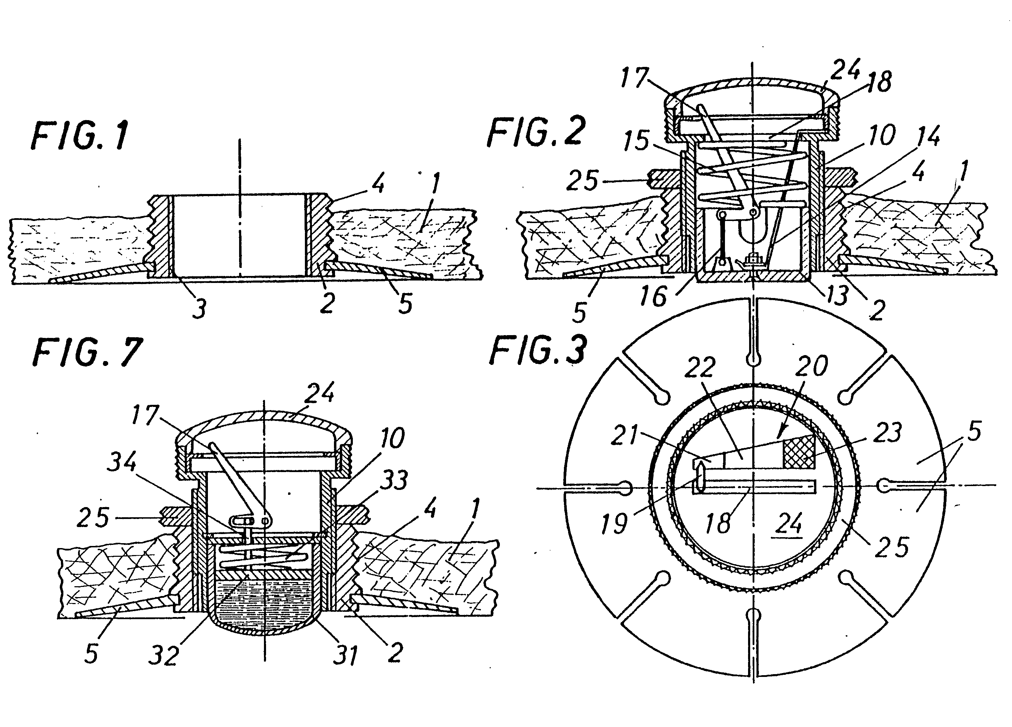

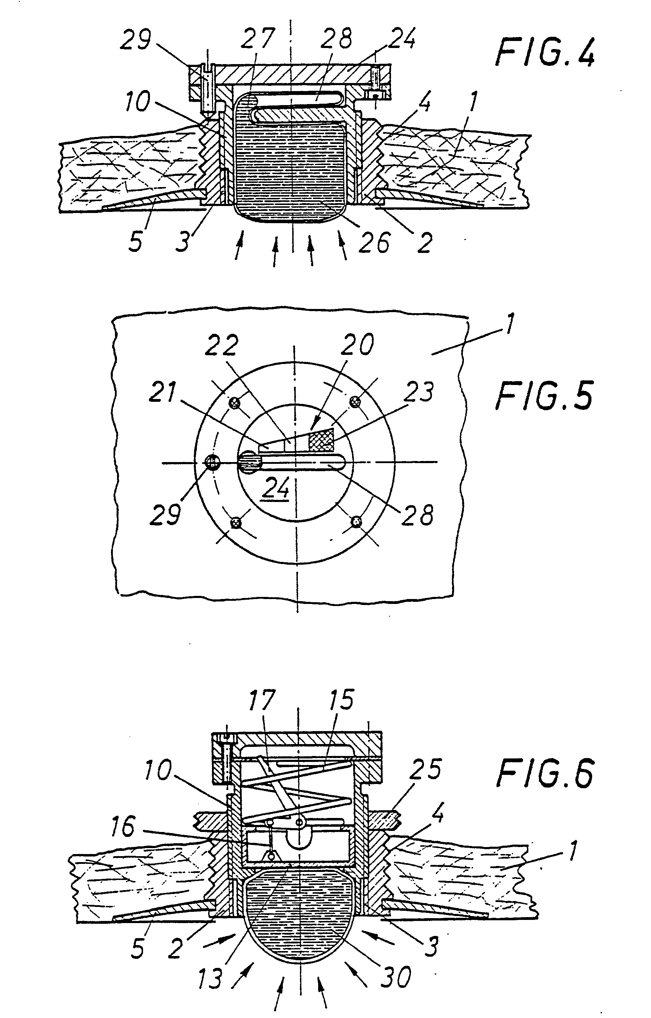

In der Zeichnung ist die Erfindung anhand von Ausführungsbeispielen schematisch dargestellt. Fig. 1 zeigt in einem Längsschnitt den in einem Stützverband verankerten Haltekörper einer erfindungsgemäßen Vorrichtung. Fig. 2 stellt eine Ausführungsform einer in einem Stützverband verankerten erfindungsgemäßen Vorrichtung dar. Fig. 3 zeigt eine Draufsicht auf Fig. 2, wobei jedoch der Stützverband weggelassen ist. Fig. 4 stellt im Längsschnitt eine weitere Ausführungsform einer in einem Stützverband verankerten erfindungsgemäßen Vorrichtung dar und Fig. 5 zeigt eine Draufsicht auf Fig. 4. Fig. 6 zeigt im Längsschnitt eine Variante zur Ausführungsform nach Fig. 2. Fig. 7 zeigt eine weitere Ausführungsform der erfindungsgemäßen Vorrichtung im Längsschnitt.

In einem als Gipsverband ausgebildeten Stützverband 1 ist ein Haltekörper 2 verankert, der von einer Hülse gebildet ist, die innen mit einem Gewinde 3 versehen ist und die außen Vorsprünge 4 aufweist oder eine Rillung, Riffelung od. dgl. besitzt, welche ein unerwünschtes Verschieben des Haltekörpers 2 im erhärtenden Gipsverband 1 verhindern. Außerdem sind am unteren Ende des Haltekörpers 2 beispielsweise aus Kunststoff bestehende Flügel 5 befestigt, die einerseits gleichfalls die Verankerung des Haltekörpers 2 im Stützverband 1 verbessern, anderseits den Haltekörper 2 während des Anbringens des Gipsverbandes 1 am Körperteil zusätzlich abstützen, so daß der Haltekörper 2 seine gewünschte Lage einnimmt.

In das Gewinde 3 des Haltekörpers 2 wird entweder ein Gehäuse 10 eingeschraubt, welches Druckfühler und Druckanzeigevorrichtung aufnimmt (siehe Fig. 2 bis 6) oder ein Füllkörper, durch welchen der von der Hülse gebildete Haltekörper 2 verschlossen wird. Dieser nicht dargestellte Füllkörper in Form einer Schraube wird beim Anlegen des Gipsverbandes 1 verwendet und verhindert, daß das Gewinde 3 durch Gipsreste oder andere Ablagerungen verschmutzt wird und dadurch das Einschrauben des Gehäuses 10 verhindert wird, ein solcher Füllkörper wird aber auch dann verwendet, wenn nach dem Abklingen der Schwellung der Druckfühler und die Druckanzeigevorrichtung entfernt werden, also das Gehäuse 10 aus dem Haltekörper 2 herausgeschraubt wird.

Der Füllkörper soll bis zum unteren Ende des Haltekörpers 2 reichen, so daß das Gewinde 3 über die gesamte Länge geschützt ist und es nach dem Entfernen des Gehäuses 10 nicht durch den nun entstandenen Hohlraum zur Bildung eines Ödems kommt.

Bei der Ausführungsform nach den Fig. 2 und 3 ist der Druckfühler von einem im Gehäuse 10 verschiebbar angeordneten Einsatzteil 13 gebildet, der durch einen Faden 14 od. dgl. gegen Herausfallen gesichert ist und durch eine Feder 15 in seine Ruhestellung gedrückt ist. Der Einsatzteil 13 ist über ein Gestänge 16 mit einem Zeiger 17 verbunden, der durch einen Schlitz 18 in der Gehäusestirnseite hindurchgeführt und an seinem aus dem Schlitz 18 herausragenden Ende 19 abgewinkelt ist. Dieses abgewinkelte Ende 19 wirkt mit einer außen auf der Gehäusestirnseite angebrachten Skala 20 zusammen, die in drei Bereiche unterteilt ist, von welchen im Bereich 21 Unterdruck herrscht, was anzeigt, daß der Stützverband zu locker geworden ist, der Bereich 22 den Normaldruck begrenzt, wo also der Druck tolerierbare Werte besitzt, und der Bereich 23 den kritischen Bereich definiert, in welchem vom Arzt Vorkehrungen getroffen werden müssen.

Das Zeigerende 19 und die Skala 20 sind durch ein als Vergrößerungslinse ausgebildetes Glas 24 abgedeckt, so daß trotz kleiner Abmessungen der Vorrichtung die wiedergegebenen Druckwerte deutlich erkennbar sind.

Nach dem Anlegen und Erhärten des Gipsverbandes 1 wird, wie bereits erwähnt, der in den Haltekörper 2 eingeschraubte Füllkörper entfernt und das Gehäuse 10 eingesetzt. Dieses Gehäuse 10 wird hiebei so weit in den Haltekörper 2 eingeschraubt, bis ein minimaler Ausschlag des Zeigerendes 19 im Bereich 22 feststellbar ist. Dadurch ist die Vorrichtung einjustiert. Im Anschluß daran wird die Lage des Gehäuses 10 bei der Ausführungsform nach den Figuren 2 und 3 durch eine Gegenmutter 25 fixiert, so daß das Gehäuse nicht mehr verdreht werden kann.

Bei der Ausführungsform nach den Fig. 4 und 5 besteht der Druckfühler aus einem im Gehäuse 10 vorgesehenen elastischen Körper 26, der an der Gehäuseunterseite aus dem Gehäuse herausragt und am Körperteil anliegt und der mit einer unkomprimierbaren Flüssigkeit gefüllt ist. Der elastische Körper 26 ist über eine Leitung 27 mit einem Schauglas 28 verbunden, in welchem das Ende der Flüssigkeitssäule sichtbar ist. Das Schauglas 28 wirkt wieder mit einer Skala 20 zusammen, die die drei erwähnten Bereiche 21 bis 23 aufweist. Durch den in Richtung der Pfeile auf den elastischen Körper 26 einwirkenden Druck infolge einer Schwellung des Körperteiles ändert sich die Lage der Flüssigkeitssäule im Schauglas 28, wobei die Höhe der Druckänderung auf der Skala 20 sichtbar ist.

Auch bei der in den Fig. 4 und 5 dargestellten Vorrichtung erfolgt deren Justierung dadurch, daß das Gehäuse 10 soweit in den Haltekörper eingeschraubt wird, daß das Ende der Flüssigkeitssäule gerade im Bereich 22, der Skala 20 sichtbar wird. Die Fixierung dieser eingestellten Lage des Gehäuses erfolgt bei der Ausführungsform nach den Fig. 4 und 5 nicht durch eine Gegenmutter, sondern durch eine stirnseitig in das Gehäuse eingeschraubte und mit der Stirnseite des Haltekörpers 2 zusammenwirkende Fixierschraube 29.

Bei der Ausführungsform nach den Fig. 2 und 3 kann es vorkommen, daß dann, wenn der Druck nicht in Verschieberichtung des Einsatzteiles 13 auf diesen einwirkt, sich der Einsatzteil 13 im Gehäuse verklemmt und infolgedessen unrichtige Druckwerte angezeigt werden. Um dies zu vermeiden, wird bei der Ausführungsform nach Fig. 6 der Einsatzteil 13 über einen elastischen Körper 30 beaufschlagt, der wieder mit einem unkomprimierbaren Medium gefüllt ist. Diese Ausführungsform gewährleistet, daß aus verschiedenen Richtungen auf den elastischen Körper 30 einwirkende Drücke in Verschieberichtung auf den Einsatzteil 13 übertragen werden und daher ein Verklemmen dieses Einsatzteiles bzw. eine falsche Druckanzeige verhindert wird.

Anstelle der Feder 15 kann auch der Einsatzteil durch ein Druckmittel hydraulisch in seine Ruhestellung gedrückt werden.

Bei der Ausführungsform nach Fig. 7 besteht der Druckfühler aus einem Zylinder 31, der mit einer elastischen bzw. nachgiebigen Wand am Körperteil anliegt und mit einer unkomprimierbaren Flüssigkeit gefüllt ist. In diesem Zylinder befindet sich ein Kolben 32, dessen eine Seite von der unkomprimierbaren Flüssigkeit und dessen andere Seite von einer Feder 33 beaufschlagt ist. Die über eine Öffnung des Zylinders 31 herausgeführte Kolbenstange 34 ist über eine mechanische Anordnung wie beim Ausführungsbeispiel nach den Fig. 2 und 3 mit dem Zeiger 17 verbunden, der wieder durch den Schlitz 18 in der Gehäusestirnseite hindurchgeführt und an seinem aus den Schlitz 18 herausragenden abgewinkelten Ende 19 mit der Skala 20 zusammenwirkt.

Es ist auch eine Ausführungsform möglich, bei welcher der Zylinder 31 keine elastische bzw. nachgiebige Wand aufweist, welche am Körperteil anliegt, sondern vielmehr der mit der unkomprimierbaren Flüssigkeit gefüllte Zylinderraum über eine Leitung mit dem bei der Ausführungsform nach den Fig. 4 ·und 5 vorhandenen elastischen Körper 26 verbunden ist. Bei dieser Ausführungsform tritt somit anstelle des bei der Ausführungsform nach den Fig. 4 und 5 angeordneten Schauglases 28 der über den Kolben 32 bzw. die Kolbenstange 34 mechanisch betätigte Zeiger 17.

**Patentansprüche**

1. Vorrichtung zum Feststellen des Druckes zwischen einem Stützverband (1) und einem von diesem Stützverband umgebenen Körperteil, mit einem an diesem Körperteil anlegbaren und am Stützverband abgestützten Druckfühler (13 ; 26 ; 30), der mit einer Druckanzeigevorrichtung gekuppelt ist, dadurch gekennzeichnet, daß der Druckfühler (13 ; 26 ; 30) und vorzugsweise auch die Druckanzeigevorrichtung (19 ; 20 ; 28) in einem Gehäuse (10) angeordnet sind, welches in einem im Stützverband (1) unverrückbar verankerbaren Haltekörper (2) entfernbar eingesetzt ist, und daß der am Körperteil unmittelbar oder mittelbar anlegbare Druckfühler (13 ; 26 ; 30) so ausgebildet ist, daß der Druck mechanisch (16) und/oder mittels eines Druckmittels (27) auf die Druckanzeigevorrichtung (19 ; 20 ; 28) übertragbar ist.

2. Vorrichtung nach Anspruch 1, dadurch gekennzeichnet, daß der Haltekörper (2) an seiner Außenseite mit Vorsprüngen (4), wie Rippen, Flügeln (5) od. dgl. versehen ist.

3. Vorrichtung nach einem der Ansprüche 1 oder 2, dadurch gekennzeichnet, daß der Haltekörper (2) von einer Hülse gebildet ist, die zumindest teilweise mit einem Innengewinde (3) versehen ist, und daß das Gehäuse (10) zylindrisch ausgebildet ist und ein dem Innengewinde (3) der Hülse entsprechendes Außengewinde aufweist.

4. Vorrichtung nach Anspruch 3, dadurch gekennzeichnet, daß auf das Gehäuse (10) eine Gegenmutter (25) zur Fixierung seiner Lage in Bezug auf die Hülse (2) aufgeschraubt ist.

5. Vorrichtung nach einem der Ansprüche 1 bis 3, dadurch gekennzeichnet, daß im Gehäuse (10) und/oder in der Hülse (2) eine Fixierschraube (29) vorgesehen ist.

6. Vorrichtung nach einem der Ansprüche 1 bis 5, dadurch gekennzeichnet, daß im Haltekörper (2) anstelle des Gehäuses (10) ein Füllkörper eingesetzt ist.

7. Vorrichtung nach einem der Ansprüche 1 bis 6, dadurch gekennzeichnet, daß der Druckfühler von einem im Gehäuse verschiebbar angeordneten Einsatzteil (13) gebildet ist, der über ein Gestänge (16) od. dgl. mit einem Zeiger (17) verbunden ist, der zusammen mit einer Skala (20) die Druckanzeigevorrichtung bildet.

8. Vorrichtung nach Anspruch 7, dadurch gekennzeichnet, daß der verschiebbar angeordnete Einsatzteil (13) durch eine Feder (15) od. dgl. in seine Ruhestellung gedrückt ist.

9. Vorrichtung nach Anspruch 7, dadurch gekennzeichnet, daß der verschiebbar angeordnete Einsatzteil (13) durch ein Druckmittel hydraulisch in seine Ruhestellung gedrückt ist.

10. Vorrichtung nach Anspruch 7, dadurch gekennzeichnet, daß der verschiebbar angeordnete Einsatzteil (13) mit einem mit einem unkomprimierbaren Medium gefüllten elastischen Körper (30) in Wirkverbindung steht, der am Körperteil anliegt.

11. Vorrichtung nach einem der Ansprüche 1 bis 6, dadurch gekennzeichnet, daß im Gehäuse (10) ein teilweise am Körperteil anliegender elastischer Körper (26) angeordnet ist, der mit einem unkomprimierbaren Medium gefüllt ist und über eine Leitung (27) mit der Druckanzeigevorrichtung verbunden ist.

12. Vorrichtung nach Anspruch 11, dadurch gekennzeichnet, daß die Druckanzeigevorrichtung aus einem mit der Leitung (27) verbundenen Schauglas (28) und einer neben dem Schauglas (28) angeordneten Skala (20) besteht.

13. Vorrichtung nach einem der Ansprüche 1 bis 11, dadurch gekennzeichnet, daß ein in einem zumindest teilweise mit einem unkomprimierbaren Medium gefüllten Zylinder (31) angeordneter Kolben (32) vorgesehen ist, dessen Kolbenstange (34) mit einem Zeiger (17) in Verbindung steht, der mit einer Skala (20) zusammenwirkt.

14. Vorrichtung nach einem der Ansprüche 7 bis 13, dadurch gekennzeichnet, daß die Skala (20) in drei Bereiche (21, 22, 23) unterteilt ist.

15. Vorrichtung nach einem der Ansprüche 7 bis 14, dadurch gekennzeichnet, daß die Skala (20) durch eine Vergrößerungslinse (24) abgedeckt ist.

**Claims**

1. Apparatus for determining the pressure between a support dressing (1) and a portion of the body surrounded by this support dressing, comprising a pressure sensor (13 ; 26 ; 30) abutted against the support dressing and being adapted to be applied to this body portion and being coupled with a pressure indicating means, characterized in that the pressure sensor (13 ; 26 ; 30) and preferably also the pressure indicating means (19 ; 20 ; 28) are positioned within a housing (10) which is removably inserted into a retaining body (2) imovably anchored in the support dressing (1), and that the pressure sensor (13 ; 26 ; 30) being directly or indirectly applyable to the body portion is so developed that the pressure can be transferred mechanically (16) and/or by means of a pressure medium (27) onto the pressure indicating means (19 ; 20 ; 28).

2. Apparatus according to claim 1, characterized in that the retaining body (2) is provided on its outer side with projections (4), such as ribs, wings (5) or the like.

3. Apparatus according to claim 1 or 2, characterized in that the retaining body (2) is formed by a sleeve provided at least partially with internal threads (3), and the housing (10) is formed cylindrically and has external threads corresponding to the internal threads (3) of the sleeve.

4. Apparatus according to claim 3, characterized in that a lock nut (25) is screwed onto the housing (10) for fixing its position relative to the sleeve (2).

5. Apparatus according to anyone of claims 1 to 3, characterized in that a set screw (29) is provided in the housing (10) and/or in the sleeve (2).

6. Apparatus according to anyone of claims 1 to 5, characterized in that a filling body is inserted in the retaining body (2) instead of the housing (10).

7. Apparatus according to anyone of claims 1 to 6, characterized in that the pressure sensor is formed by an insert element (13) slidably arranged within the housing and being connected by means of a lever mechanism (16) or the like with a pointer (17) which together with a graduation (20) forms the pressure indicating means.

8. Apparatus according to claim 7, characterized in that the slidably arranged insert element (13) is urged by a spring (15) or the like into its inoperative position.

9. Apparatus according to claim 7, characterized in that the slidably arranged insert element (13) is urged hydraulically by a pressure medium into its inoperative position.

10. Apparatus according to claim 7, characterized in that the slidably arranged insert element (13) is operatively connected to a resilient body (30) filled with an incompressible medium, which body abuts against the body portion.

11. Apparatus according to anyone of claims 1 to 6, characterized in that a resilient body (26) partially abutting against the body portion is positioned in the housing (10), which body is filled with an incompressible medium and is connected by a conduit (27) to the pressure indicating means.

12. Apparatus according to claim 11, characterized in that the pressure indicating means consists of a sight glass (28) connected to the conduit (27) and of a graduation (20) positioned beside the sight glass (28).

13. Apparatus according to anyone of claims 1 to 11, characterized in that a piston (32) is provided in a cylinder (31) filled at least partially with an incompressible medium, the piston rod (34) of which piston is connected to a pointer (17) cooperating with a graduation (20).

14. Apparatus according to anyone of claims 7 to 13, characterized in that the graduation (20) is divided into three ranges (21, 22, 23). ·

15. Apparatus according to anyone of claims 7 to 14, characterized in that the graduation (20) is covered by a magnifying lens (24).

## Revendications

1. Appareil pour déterminer la pression entre un bandage contentif (1) et une partie du corps entourée de ce bandage contentif, muni d'un capteur de pression (13 ; 26 ; 30) ·pouvant être appliqué à cette partie du corps et appuyé sur le bandage contentif et qui est accouplé à un dispositif d'affichage de pression, caractérisé en ce que le capteur de pression (13 ; 26 ; 30) et, de préférence, aussi le dispositif d'affichage de pression (19 ; 20 ; 28) sont disposés dans un boîtier (10) qui est inséré de façon amovible dans un corps de retenue (2) pouvant être ancré de façon inébranlable dans le bandage contentif (1), et en ce que le capteur de pression (13 ; 26 ; 30) pouvant être appliqué directement ou indirectement à la partie du corps est constitué de telle sorte que la pression peut être transmise mécaniquement (16) et/ou au moyen d'un agent de pression (27) au dispositif d'affichage de pression (19 ; 20 ; 28).

2. Appareil selon la revendication 1, caractérisé en ce que le corps de retenue (2) est muni à son côté extérieur de saillies (4) telles que des nervures, des ailettes (5), ou analogues.

3. Appareil selon l'une des revendications 1 et 2, caractérisé en ce que le corps de retenue (2) est formé par une douille qui est munie au moins partiellement d'un filetage intérieur (3), et en ce que le boîtier est constitué sous forme cylindrique et présente un filetage extérieur correspondant au filetage intérieur (3) de la douille.

4. Appareil selon la revendication 3, caractérisé en ce que sur le boîtier (10) est vissé un contre-écrou (25) pour la fixation de sa position relativement à la douille (2).

5. Appareil selon l'une des revendications 1 à 3, caractérisé en ce que dans le boîtier (10) et/ou dans la douille (2) est prévue une vis de fixation (29).

6. Appareil selon l'une des revendications 1 à 5, caractérisé en ce que dans le corps de retenue (2) est inséré, au lieu du boîtier (10), un corps de remplissage.

7. Appareil selon l'une des revendications 1 à 6, caractérisé en ce que le capteur de pression est formé par un élément d'insertion (13) disposé dans le boîtier de manière à pouvoir coulisser, qui est relié par l'intermédiaire d'un tringlage (16), ou analogue, à une aiguille (17) qui forme, en même temps qu'une échelle (20), le dispositif d'affichage de pression.

8. Appareil selon la revendication 7, caractérisé en ce que l'élément d'insertion (13) disposé de manière à pouvoir coulisser est poussé à sa position de repos par un ressort (15), ou analogue.

9. Appareil selon la revendication 7, caractérisé en ce que l'élément d'insertion (13) disposé de manière à pouvoir coulisser est poussé hydrauliquement à sa position de repos par un agent de pression.

10. Appareil selon la revendication 7, caractérisé en ce que l'élément d'insertion (13) est en liaison fonctionnelle avec un corps élastique (30) rempli d'un milieu incompressible et qui s'applique à la partie du corps.

11. Appareil selon l'une des revendications 1 à 6, caractérisé en ce que dans le boîtier (10) est disposé un corps élastique (26) s'appliquant partiellement à la partie du corps et qui est rempli d'un milieu incompressible et est relié par l'intermédiaire d'un tuyau (27) au dispositif d'affichage de pression.

12. Appareil selon la revendication 11, caractérisé en ce que le dispositif d'affichage de pression est formé d'un verre-regard (28) relié au tuyau (27) et d'une échelle (20) disposée à côté du verre-regard (28).

13. Appareil selon l'une des revendications 1 à 11, caractérisé en ce qu'il est prévu un piston (32) disposé dans un cylindre au moins partiellement rempli d'un milieu incompressible et dont la tige de piston (34) est en liaison avec l'aiguille (17) qui coopère avec une échelle (20).

14. Appareil selon l'une des revendications 7 à 13, caractérisé en ce que l'échelle (20) est subdivisée en trois régions (21, 22, 23).

15. Appareil selon l'une des revendications 7 à 14, caractérisé en ce que l'échelle (20) est recouverte par une lentille grossissante (24).

FIG.1

FIG.2

FIG.7

FIG.3

0 088 860

FIG.4

FIG.5

FIG.6